# EUROPEAN PATENT SPECIFICATION

(11) **EP 1 479 405 B1**
(45) Date of publication and mention of the grant of the patent: **19.07.2006**
(21) Application number: 04252988.3
(22) Date of filing: 21.05.2004
(51) Int. Cl.: A61M 16/04, A61M 39/10, A61M 39/00, A61M 16/00

(54) **One touch connector for tracheotomy tube**
Mit einem Griff verbindbare Verbindungseinrichtung für eine Luftröhrenkanüle
Connecteur à touche unique pour un tube de trachéostomie

(30) Priority: 21.05.2003 JP 2003143104; 27.04.2004 JP 2004130729
(43) Date of publication of application: 24.11.2004
(73) Proprietor: KABUSHIKI KAISHA TOP, Tokyo 120-0035 (JP)
(72) Inventor: Kawahara, Keiji, Tokyo 120-0035 (JP); Ichikawa, Kazuhiro, Tokyo 120-0035 (JP); Araki, Tetsuro Araki Industrial Design Corp., Numazu-shi Shizuoka 410-0054 (JP)
(74) Representative: Fenlon, Christine Lesley

(56) References cited:
- EP-A- 0 471 574
- EP-A- 0 753 323
- EP-A- 0 768 096
- WO-A-95/04566
- US-A- 4 639 019
- US-A- 5 259 376
- US-A1- 2002 029 782

## Description

The present invention relates to a one touch connector for a tracheotomy tube, and more specifically, to a connector that connects an end of a tracheotomy tube to an end of another tube extended from an artificial respiration circuit.

A connector according to the preamble of claim 1 is disclosed in WO-A-95/04566.

In general, a tracheotomy tube comprises a male terminal portion at one end to which a female terminal portion provided at an end of a tube from an artificial respiration circuit is connected.

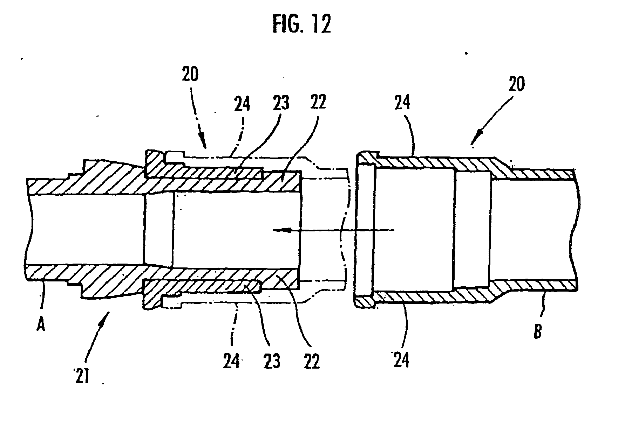

In the prior art, a tracheotomy tube of this kind is brought into a connected state by inserting a male terminal portion 21 into a female terminal portion 20 as shown in FIG. 12. The male terminal portion 21 comprises a cylindrical member 23 made of a synthetic resin and fitted and held in an end of a tube main body 22 made of a rubber-like elastomer or a soft elastomer. The female terminal portion 20 is formed at an end of a tube main body 24 made of a synthetic resin. The female terminal portion 20 is formed so as to engage with the cylindrical member 23 utilizing their taper shapes. Thus, when the male terminal portion 21 and the female terminal portion 20 are connected together, an inner surface of the female terminal portion 20 is brought into pressure contact with an outer surface of the cylindrical member 23 of the male terminal portion 21 for firm engagement.

The tube from the artificial respiration circuit must be very promptly connected to and removed from the tracheotomy tube. However, when the male terminal portion 21 and the female terminal portion 20 are connected together very promptly, they are prone to be inadequately connected. Consequently, the male terminal portion 21 may inadvertently slip out of the female terminal portion 20 or air may leak from the connection between the female terminal portion 20 and the male terminal portion 21.

Further, to form a reliable connected state, the male terminal portion 21 is preferably inserted deeper into the female terminal portion 20 for a firm connection. However, the firmly connected male terminal portion 21 and female terminal portion 20 are very difficult to remove from each other. Thus, when the male terminal portion 21 is removed from the female terminal portion 20, or vice versa, the male terminal portion 21, connected to the tube from the artificial respiration circuit, must be pulled forcefully. Then, the tracheotomy tube is correspondingly pulled to make the patient with the tracheotomy tube feel pain. Furthermore, when the tracheotomy tube main body is attached to the patient, the female terminal portion 20 is located close to the patient's neck. Accordingly, a space in the tracheotomy tube main body which is available for gripping is very small. If the male terminal portion 21 and the female terminal portion 20 are pulled from each other by gripping both of them, it is difficult to hold the tracheotomy tube main body. A removing operation is thus very difficult to perform. Further, if the male terminal portion 21 and the female terminal portion 20 are removed from each other, for example, a U-shaped removing instrument may be interposed between the male terminal portion 21 and the female terminal portion 20 to clear their fitting. However, the removing instrument is separate from the tracheotomy tube and is thus likely to be lost. Consequently, the removing instrument is cumbersome to handle. Thus, in the prior art, the artificial respiration circuit can disadvantageously not be removed from the tracheotomy tube promptly.

Preferred embodiments of the present invention seek to eliminate the above disadvantages to provide a one touch connector for a tracheotomy tube which can firmly maintain the connection of the tracheotomy tube and which enables the smooth disconnection of the tracheotomy tube.

Preferred embodiments of the present invention provide a one touch connector for a tracheotomy tube which connects a male terminal portion provided at a leading end of the tracheotomy to a female terminal portion provided at a leading end of a respiration circuit terminal, wherein the male terminal portion comprise a tapered outer peripheral engagement surface formed around an outer periphery of the male terminal portion and an annular convex portion projecting like a flange along a trailing edge of the outer peripheral engagement surface. The female terminal portion preferably comprises a tapered inner peripheral engagement surface formed around an inner periphery of the female terminal portion and engaged in pressure contact with the outer peripheral engagement surface. Móreover, the female terminal portion preferably has an external cylinder installed around an outer periphery of the female terminal portion and formed of an elastic and flexible material.

The external cylinder preferably comprises a pair of first opposite wall portions that lie opposite each other via the female terminal portion and at a predetermined distance from the female terminal portion. A pair of second opposite wall portions are preferably connected to the respective first opposite wall portions so as to be connected to the first opposite wall portions and lying opposite each other via the female terminal portion, and preferably close to the female terminal portion. The pair of second opposite wall portions being advantageously snappily flexed and separated from the female terminal portion when both first opposite wall portions are pressed in a direction in which the first opposite wall portions near each other.

Each of the second opposite wall portions preferably comprises a locking pawl projected from an inner surface of the second opposite wall portion and being locked on a rear side wall of the annular convex portion when the outer peripheral engagement surface is engaged in pressure contact with the inner peripheral engagement surface, the locking pawl preferably being unlocked from the rear side wall of the annular convex portion when both first opposite wall portions are pressed to snappily flex the second opposite wall portions.

Each of the first opposite wall portions preferably comprises a pressing pawl that is projected from an inner surface of the first opposite wall portions and preferably abuts against a front side wall of the annular convex portion when the pressing operation is performed, to press the male terminal portion via the annular convex portion in a direction in which the male terminal portion slips out, thus disengaging the outer peripheral engagement surface from the inner peripheral engagement surface.

According to an embodiment of the present invention, when the male terminal portion is inserted into the female terminal portion, the inner peripheral engagement surface of the female terminal portion is engaged in pressure contact with the outer peripheral engagement surface of the male terminal portion. The male terminal portion is preferably inserted deep into the female terminal portion. Then, at the same time when the male terminal portion is connected to the female terminal portion in an adequate engaged state, the locking pawls, formed on the second opposite wall portions of the external cylinder, are preferably locked, on the rear side wall of the annular convex portion. This surely prevents the male terminal portion from slipping out of the female terminal portion. An adequate connected state can be rigidly maintained.

To disconnect the female terminal portion from the male terminal portion, the first opposite wall portions are pressed so as to come near each other. Thus, both second opposite wall portions are snappily flexed in a direction in which they are separated from the female terminal portion. This unlocks the locking pawls from the rear side wall of the annular convex portion. At the same time, the first opposite wall portions are pressed to come near the female terminal portion. The pressing pawls abut against the front side wall of the annular convex portion. The abutment of the pressing pawls against the front side wall pushes the male terminal portion out in the direction in which it slips out of the female terminal portion. This loosens the firm pressure contact engagement between the outer peripheral engagement surface of the male terminal portion and the inner peripheral engagement surface of the female terminal portion. Consequently, the female terminal portion can be smoothly disconnected from the male terminal portion.

The female terminal portion can be disconnected from the male terminal portion simply by pressing the first opposite wall portions. Accordingly, the male terminal portion, connected to another tube, need not be pulled forcefully. Further, the female terminal portion can be disconnected from the male terminal portion without the need to grip the tracheotomy tube main body, or even if the tracheotomy tube must be gripped, the disconnection can be accomplished simply by softly touching the tracheotomy tube. Thus, the female terminal portion can be smoothly disconnected from the male terminal portion without making the patient with the tracheotomy tube feel pain and even if the tracheotomy tube main body has only a very small space available for gripping. Furthermore, the female terminal portion can be very easily disconnected from the male terminal portion without using any U-shaped removing instruments as in the case of the prior art. Thus, embodiments of the present invention make it possible to accomplish both firm connection between the male terminal portion and the female terminal portion and easy removal of the male terminal portion from the female terminal portion. Preferred embodiments of the present invention also enable a quick connection and disconnection between the female terminal portion and the male terminal portion.

Further, the external cylinder preferably comprises a fitting portion around an inner periphery of the external cylinder, the fitting portion being rotatably fitted into a groove portion formed all along the outer periphery of the female terminal portion. The external cylinder is rotatably provided around the outer periphery of the female terminal portion via the fitting portion. Accordingly, the external cylinder can be rotated to turn both first opposite wall portions so that they lie in the direction in which they can be pressed more easily. Thus, the first opposite wall portions can be easily pressed to smoothly connect and disconnect the male terminal portion to and from the female terminal portion.

Furthermore, preferred embodiments of the present invention may further comprise a regulating member which is removably provided between the outer periphery of the female terminal portion and each first opposite wall portion and which, when inserted between the outer periphery of the female terminal portion and each first opposite wall portion, regulates the flexure of both first opposite wall portions in which the first opposite wall portions come near each other, while maintaining the locking of the locking pawls on the annular convex portion.

According to an embodiment of the present invention, by inserting the regulating member between the outer periphery of the female terminal portion and each first opposite wall portion, the flexure of both first opposite wall portions and both second opposite wall portions is regulated even if both first opposite wall portions are inadvertently pressed in the direction in which they come near each other. Accordingly, the locking of the locking pawls on the annular convex portion is maintained to surely prevent the male terminal portion from slipping out of the female terminal portion. Therefore, an adequate connected state can be tightly maintained.

In this case, the regulating member is preferably formed like rings and rotatably installed on the outer periphery of the female terminal portion, and comprises a pair of regulating portions formed to be thick enough to regulate the flexure of both first opposite wall portions in the direction in which the first opposite wall portions come near each other, when the regulating member is rotated in one direction to insert each regulating portion between the outer periphery of the female terminal portion and the corresponding first opposite wall portion, and deregulating portions each connected to both regulating portions so as to be connected to the regulating portions and formed to be thinner than the regulating portions, the deregulating portions allowing both first opposite wall portions to freely flex in the direction in which the first opposite wall portions come near each other, when the regulating member is rotated in the other direction to place each deregulating portion between the outer periphery of the female terminal portion and the corresponding first opposite wall portion.

Thus, simple rotation of the ring-like regulating member enables regulation and deregulation of flexure of both first opposite wall portions. Specifically, simply by rotating the ring-like regulating member in one direction after the male terminal portion has been inserted into the female terminal portion for connection, it is possible to position the regulating portion between the outer periphery of the female terminal portion and each first opposite wall portion. This enables a firm connection to be promptly made and maintained between the male terminal portion and the female terminal portion. Further, to disconnect the female terminal portion from the male terminal portion, the ring-like regulatingmember may simply be rotated in the other direction before the first opposite wall portions are pressed. Then, each deregulating portion can be positioned between the outer periphery of the female terminal portion and the corresponding first opposite wall portion. Therefore, the female terminal portion can be promptly disconnected from the male terminal portion.

The one touch connector for a tracheotomy tube according to the present invention can similarly be employed for a button type gastric fistula tube, an artificial anus, and other tubes having a connector close to the human body. If the female terminal portion is attached to the human body, it is possible to provide an annular convex portion in the female terminal, while providing the external cylinder and regulating member

For a better understanding of the present invention, and to show how the same may be carried into effect, reference will now be made, by way of example to the accompanying drawings in which:
FIG. 1 is a perspective view illustrating a connector according to a first embodiment;
FIG. 2A is a front view illustrating an external cylinder of the connector according to the first embodiment;
FIG. 2B is a view illustrating how the external cylinder in FIG. 2A is pressed and deformed;
FIG. 3 is a sectional view taken along a line III-III in FIG. 2A:
FIG. 4 is a sectional view taken along a line IV-IV in FIG. 2A;
FIG. 5A is a sectional view illustrating first opposite wall portions and pressing pawls during a connecting operation of a connector according to a first embodiment;
FIG. 5B is a sectional view illustrating the first opposite wall portions and the pressing pawls during a removing operation of a connector according to a first embodiment:
FIG. 6A is a sectional view illustrating second opposite wall portions and locking pawls during a connecting operation of a connector according to a first embodiment;
FIG. 6B is a sectional view illustrating the second opposite wall portions and the locking pawls during a removing operation of a connector according to a first embodiment;
FIG. 7 is a perspective view illustrating a connector according to a second embodiment;
FIG. 8A is a front view illustrating an external cylinder of the connector according to the second embodiment;
FIG. 8B is a view illustrating how the external cylinder in FIG. 8A is pressed and deformed:
FIG. 9 is a view illustrating a regulating portion;
FIG. 10A is sectional view illustrating first opposite wall portions and pressing pawls during a connecting operation of a connector according to a first embodiment;
FIG. 10B is a sectional view illustrating the first opposite wall portions and the pressing pawls during a removing operation of a connector according to a first embodiment;
FIG. 11A is a sectional view illustrating second opposite wall portions and locking pawls during a connecting operation of a connector according to a first embodiment;
FIG. 11B is a sectional view illustrating the second opposite wall portions and the locking pawls during a removing operation of a connector according to a first embodiment; and
FIG. 12 is a sectional view illustrating a connection of the tracheotomy tube..

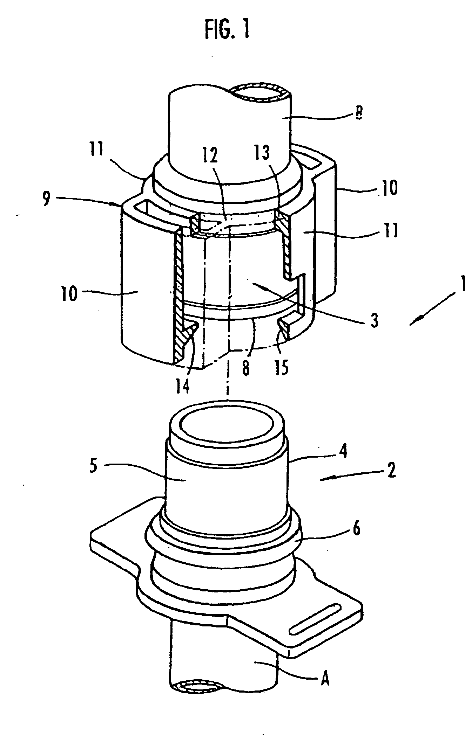

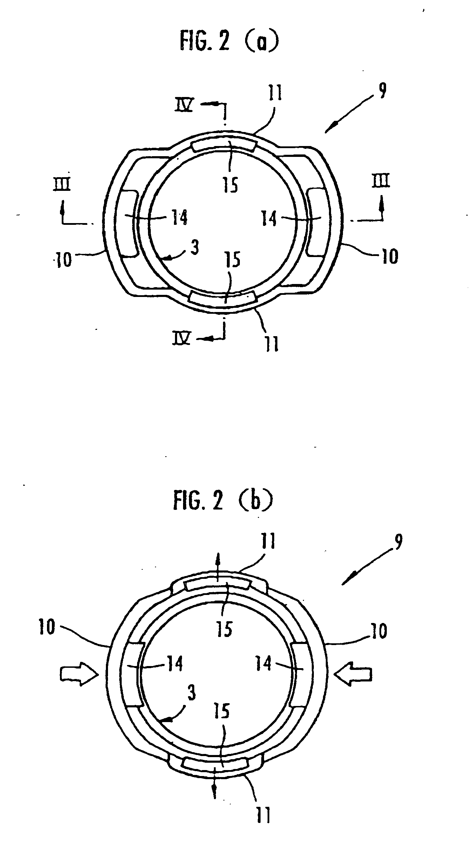

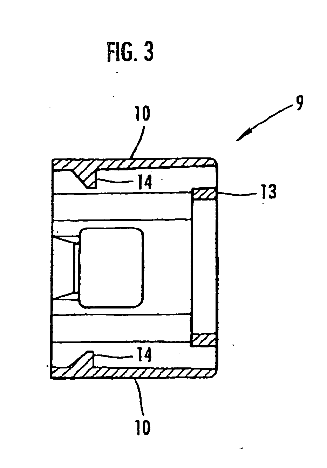

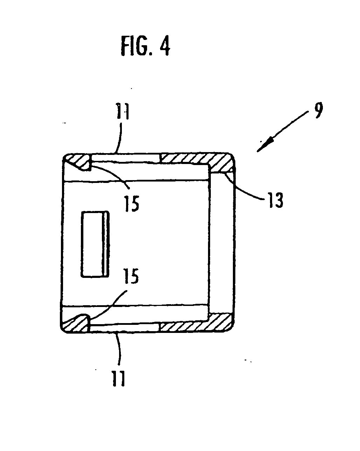

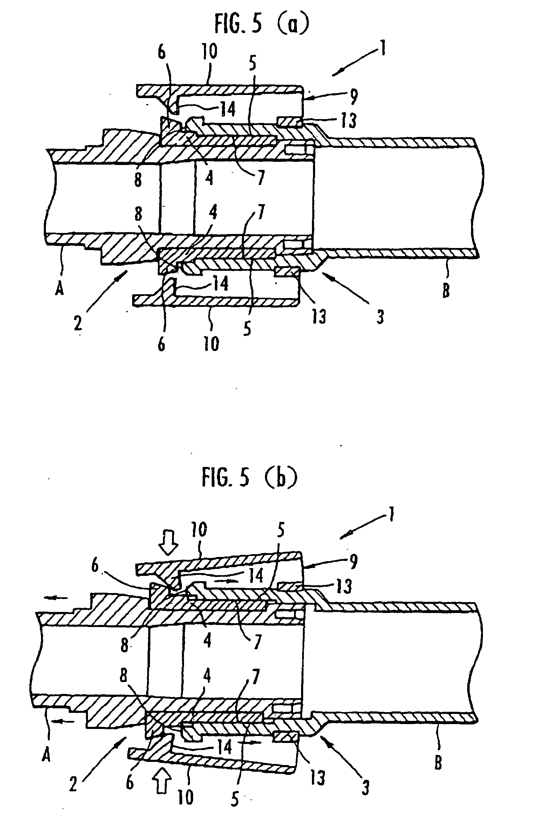

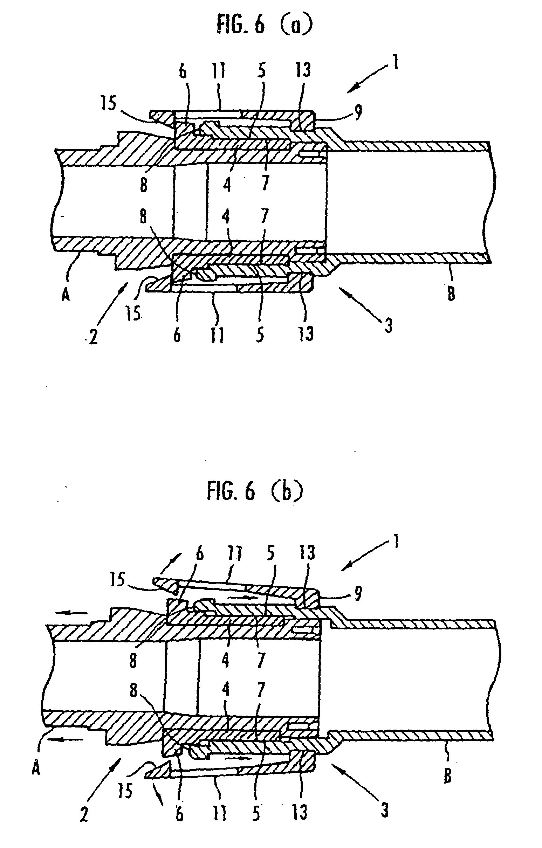

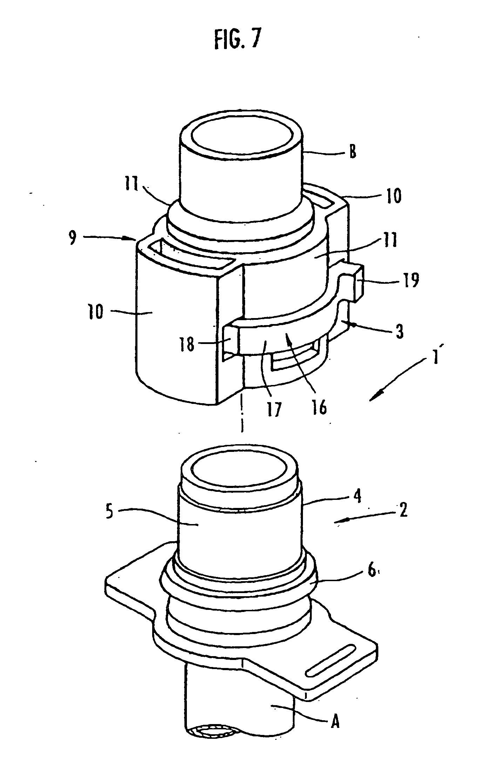

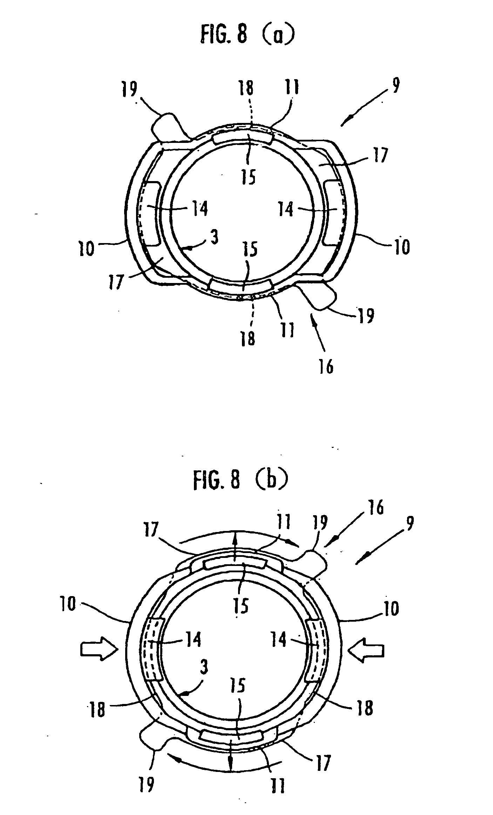

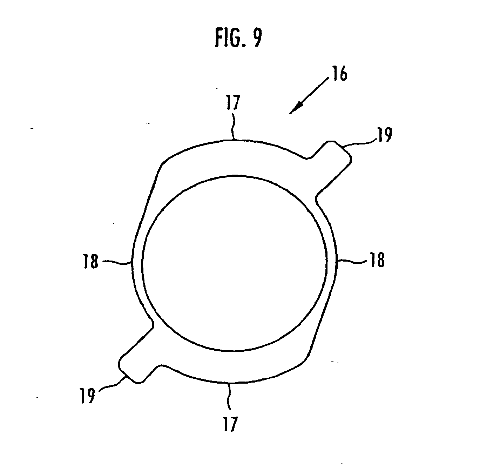

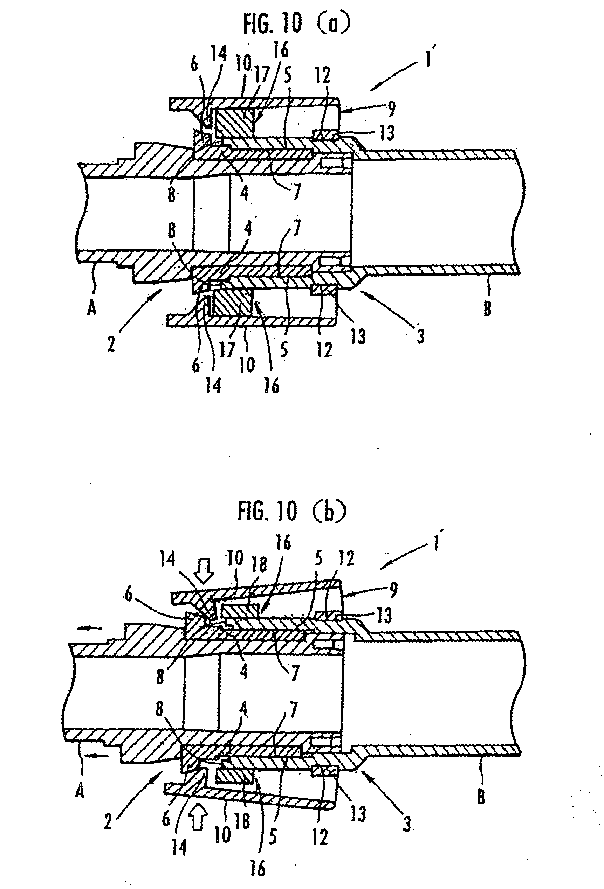

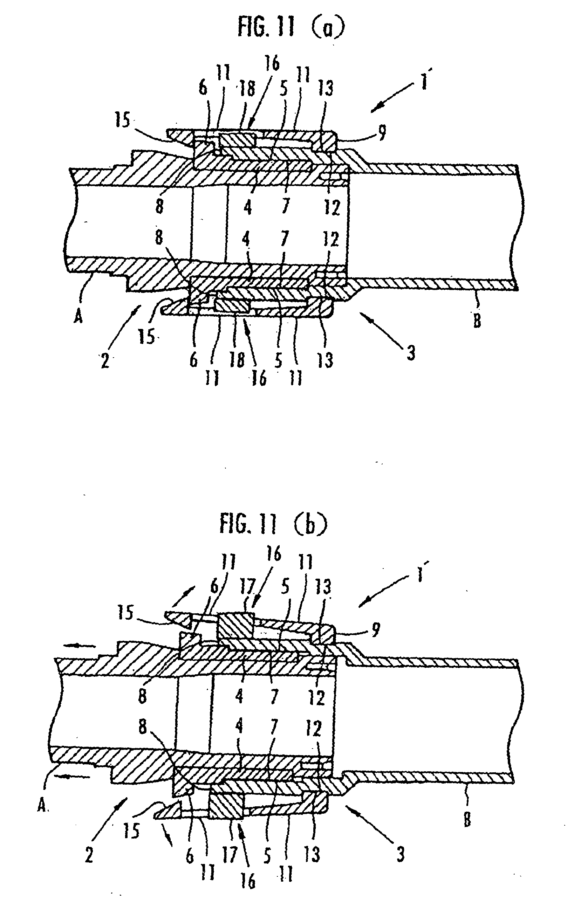

FIG. 1 is a perspective view illustrating a connector according to a first embodiment. FIG. 2A is a front view illustrating an external cylinder of the connector according to the first embodiment. FIG. 2B is a view illustrating how the external cylinder in FIG. 2A is pressed and deformed. FIG. 3 is a sectional view taken along a line III-III in FIG. 2A. FIG. 4 is a sectional view taken along a line IV-IV in FIG. 2A. FIG. 5A is a sectional view illustrating first opposite wall portions and pressing pawls during a connecting operation. FIG. 5B is a sectional view illustrating the first opposite wall portions and the pressing pawls during a removing operation. FIG. 6A is a sectional view illustrating second opposite wall portions and locking pawls during a connecting operation. FIG. 6B is a sectional view illustrating the second opposite wall portions and the locking pawls during a removing operation. FIG. 7 is a perspective view illustrating a connector according to a second embodiment. FIG. 8A is a front view illustrating an external cylinder of the connector according to the second embodiment. FIG. 8B is a view illustrating how the external cylinder in FIG. 8A is pressed and deformed. FIG. 9 is a view illustrating a regulating portion. FIG. 10A is sectional view illustrating first opposite wall portions and pressing pawls during a connecting operation. FIG. 10B is a sectional view illustrating the first opposite wall portions and the pressing pawls during a removing operation. FIG. 11A is a sectional view illustrating second opposite wall portions and locking pawls during a connecting operation. FIG. 11B is a sectional view illustrating the second opposite wall portions and the locking pawls during a removing operation.

First, with reference to FIGS. 1 to 6, description will be given of a one touch connector 1 for a tracheotomy tube according to a first embodiment of the present invention.

The one touch connector 1 for a tracheotomy tube according to the first embodiment of the present invention connects a circuit tube B extended from an artificial respirator circuit to a tracheotomy tube A as shown in FIG. 1. The one touch connector 1 is composed of a male terminal portion 2 provided at a leading end of the tracheotomy tube A and a female terminal portion 3 provided at a leading end of the circuit tube B.

As shown in FIGS. 1 and 5A, the male terminal portion 2 comprises a cylindrical member 4 made of a synthetic resin and rotatably provided at the leading ed of the tracheotomy tube A, formed of a rubber-like elastomer or a soft elastomer (for example, silicone rubber). The cylindrical member 4 comprises an outer peripheral engagement surface 5 around its outer periphery and an annular convex portion 6 projecting along a trailing edge of the outer peripheral engagement surface 5 like a flange.

As shown in FIGS. 1 and 5A, the female terminal portion 3 is formed around the circuit tube B formed by synthetic resin and comprises an inner peripheral engagement surface 7 engaged in pressure contact with the outer peripheral engagement surface 5.

As shown in FIG. 1, the female terminal portion 3 has an external cylinder 9 rotatably provided around its outer periphery and formed of an elastic, flexible synthetic resin. The external cylinder 9 comprises a pair of first opposite wall portions 10 that are opposite each other as shown in FIGS. 2A and 3 and a pair of second opposite wall portions 11 that are opposite each other orthogonally to the direction in which the first opposite wall portions 10 are opposite each other as shown in FIGS. 2A and 4. As shown in FIGS. 3 and 4, the external cylinder 9 also comprises a fitting portion 13 that projects inward of its trailing end and that rotatably fits into a groove portion 12 (see FIG. 1) formed all along the outer periphery of the female terminal portion 3.

The external cylinder 9 is shaped so that when the first opposite wall portions 10 of the pair are pressed in the direction in which the wall potions 10 comes near each other, the second opposite wall portions 11 of the pair are flexed in the direction in which the wall portions 11 are separated from each other as shown in FIG. 2B. Further, as shown in FIG. 3, a pressing pawl 14 having a triangular cross section is projected inward from each first opposite wall portion 10. As shown in FIG. 4, a locking pawl 15 having a triangular cross section is projected inward from each second opposite wall portion 11.

Each of the pressing pawls 14 is provided so that when the male terminal portion 2 and the female terminal portion 3 are connected together, the pressing pawl 14 faces a front side wall of an annular convex portion 6. as shown in FIG. 5A. Each of the locking pawls 15 is provided so that when the male terminal portion 2 and the female terminal portion 3 are connected together, the locking pawl 15 faces a rear side wall of the annular convex portion 6, as shown in FIG. 6A.

When the male terminal portion 2 and the female terminal portion 3 are connected together so that the former is inserted into the latter, the inner peripheral engagement surface 7 of the female terminal portion 3 is engaged in pressure contact with the outer peripheral engagement surface 5. At this time, as shown in FIG. 6A, each locking pawl 15, provided on the corresponding second opposite wall portion 11 of the external cylinder 9, is snappily locked on the rear side wall of the annular convex portion 6 of the cylindrical member 4. This prevents the male terminal portion 2 from slipping out of the female terminal portion 3. That is, the connection between the male terminal portion 2 and the female terminal portion 3 is reliably maintained.

Since the cylindrical member 4 of the male terminal portion 2 is provided so as to be freely rotatable relative to the tracheotomy tube A, the female terminal portion 3 can be rotated through a desired angle relative to the male terminal portion 2 while the inner peripheral engagement surface 7 of the female terminal portion 3 is engaged in pressure contact with the outer peripheral engagement surface 5 of the cylindrical member 4. On this occasion, the locking pawls 15 lock the rear side wall of the annular convex portion 6 of the cylindrical member 4, with the pressing pawls 14 separated from the annular convex portion 6. Accordingly, the female terminal portion 3 can be smoothly rotated relative to the male terminal portion 2. Moreover, as previously described, the external cylinder 9 is provided so as to be freely rotatable all along the outer periphery of the female terminal portion 3 via the fitting portion 13. Accordingly, the external cylinder 9 can be rotated to turn both first opposite wall portions 10 so that they lie in the direction in which they are more easily pressed.

To disconnect the male terminal portion 2 from the female terminal portion 3, both first opposite wall portions 10 of the external cylinder 9, in the connected state shown in FIGS. 5A and 6A, are pressed in the direction in which they come near each other as shown in FIG. 2A. Thus, both second opposite wall portions 11 are flexed outward to separate the locking pawls 15 from the rear side wall of the annular convex portion 6 of the cylindrical member 4 as shown in FIG. 6B. At the same time, since the both first opposite wall portions 10 of the external cylinder 9 have been pressed, the pressing pawls 14 abut against the front side wall of the annular convex portion 6 of the cylindrical member 4 of the male terminal portion 2 to push out the male terminal portion 2 in the direction
in which it slips out of the female terminal portion as shown in FIG. 5B. This weakens the pressure contact engagement between the inner peripheral engagement surface 7 of the female terminal portion 3 and the outer peripheral surface 5 of the cylindrical member 4 of the male terminal portion 2. Consequently, the male terminal portion 2 is easily disconnected from the female terminal portion 3.

As described above, with the one touch connector 1 for a tracheotomy tube according to the present embodiment, the locking pawls 15 of the external cylinder 9 are locked on the annular convex portion 6 of the cylindrical member 4 when the male terminal portion 2 is reliably connected to the female terminal portion 3 so that the former is inserted into the latter. This enables the maintenance of a connected state
in which the male terminal portion 2 is surely prevented from slipping out of the female terminal portion 3. Further, simply by pressing the first opposite wall portions 10 of the external cylinder 9 in the direction in which they come near each other, the locking pawls 15 are unlocked from the annular convex portion 6, while the pressing pawls 14 push out the male terminal portion 2 via the annular convex portion 6. Thus, the tight engagement is loosened to enable the female terminal portion 3 to be smoothly disconnected from the male terminal portion 2. In this way, only pressing of the first opposite wall portion 10 provides release of the connection of the female terminal portion 3 and the male terminal portion 2. This eliminates the need to forcefully pull the male terminal portion 2. connected to the circuit tube B. The circuit tube B can be removed from the tracheotomy tube A substantially without any resistance. Moreover, the circuit tube B can be removed without the need to grip the main body of the tracheotomy tube A, or even if the tracheotomy tube A must be gripped, the disconnection can be accomplished simply by softly touching the tracheotomy tube A. Thus, the circuit tube B can be smoothly removed without making the patient feel pain and even if the tracheotomy tube A main body has only a very small space available for gripping. Furthermore, both first opposite wall portions 10 can be pressed with one hand, and the tube B can be very easily removed without using any U-shaped removing instruments (not shown) as in the case of the prior art. Thus, the present embodiment enables the tracheotomy tube A and the circuit tube B to be reliably firmly connected together and also enables the circuit tube B to be smoothly and quickly removed from the tracheotomy tube A simply by pressing both first opposite wall portion 10.

Now, with reference to FIGS. 7 to 11, description will be given of a one touch connector 1' for a tracheotomy tube according to a second embodiment of the present invention. For the description of the one touch connector 1' for a tracheotomy tube according to the second embodiment, description will be given only of those parts of the configuration of the one touch connector 1' which are different from those of the one touch connector 1 for a tracheotomy tube according to the already described first embodiment. In FIGS. 7 to 11. the same parts of the configuration of the one touch connector 1' as those of the one touch connector 1 for a tracheotomy tube according to the already described first embodiment are denoted by the same reference numerals as those in FIGS. 1 to 6.

A regulating member 16 is provided on the female terminal portion 3 of the one touch connector 1' for a tracheotomy tube according to the second embodiment as shown in FIG. 7. As shown in FIG. 9, the regulating member 16 is formed like a ring and comprises a pair of regulating portions 17 formed to be thicker and deregulating portions 18 formed to be thinner: the regulating portions 17 are connected to the deregulating portions 18. Moreover, an operating convex portion 19 projecting in an outer peripheral direction is formed at the boundary between each regulating portion 17 and the corresponding deregulating portion 18 . The regulating member 16 is rotatably supported around the female terminal portion 3 as shown in FIG. 8. When the regulating portions 17 is located inside the respective first opposite wall portions 10 as shown in FIG. 10A. the regulating member 16 regulates the flexure of the first opposite wall portions 10. When the deregulating portions 18 are located inside the respective first opposite wall portions 10 as shown in FIG. 10B, each regulating portion 17 is located under the second opposite wall portion 11 as shown in FIG. 7 to allow the first opposite wall portions 10 to be freely flexed.

With the one touch connector 1' for a tracheotomy tube according to the second embodiment, to connect the male terminal portion 2 and the female terminal portion 3 together, the operating convex portions 19 of the regulating member 16 are operated and rotated to locate each regulating portions 17 under the second opposite wall portion 11 as shown in FIG. 7. This allows the first opposite wall portions 10 to be freely flexed. Thus, when the male terminal portion 2 and the female terminal portion 3 are connected together so that the former is inserted into the latter, the inner peripheral engagement surface 7 of the female terminal portion 3 is engaged in pressure contact with the outer peripheral engagement surface 5 as shown in FIG. 11A.

Subsequently, the operating convex portion 19 of the regulating member 16 is operated and rotated to position each regulating portion 17 inside the first opposite wall portion 10 as shown in FIGS. 8A and 8B. The flexure of the first opposite wall portions 10 is thus prevented. This further prevents the male terminal portion 2 from slipping out to enable the more reliable maintenance of the connection of the male terminal portion 2 to the female terminal portion 3. Each regulating portion 17 may be formed to have a thickness dimension that allows the thirst opposite wall potions 10 to be slightly pressed so as to separate from each other when it is positioned inside the corresponding first opposite wall portion 10. In this case, the regulating portions 17 cause both first opposite wall portions 10 to be flexed in the direction in which they are separated from each other. Correspondingly, the second opposite wall portions 11 are flexed in the direction in which they come near each other. Therefore, the locking pawls 15, provided on the respective second opposite wall portions 11, can be more deeply and firmly engaged with the rear side wall of the annular convex portion 6 of the cylindrical member 4.

With the one touch connector 1' for a tracheotomy tube according to the second embodiment, to disconnect the male terminal portion 2 from the female terminal portion 3, the operating convex portion 19 of the regulating member 16 is operated and rotated from the state shown in FIG. 8A to the state shown in FIG. 8B. Then, as shown in FIG. 8B. both first opposite wall portions 10 of the external cylinder 9 are pressed in the direction in which they near each other. Thus, both second opposite wall portions 11 of the external cylinder 9 are flexed outward to separate the locking pawls 15 from the rear side wall of the annular convex portion 6 as shown in FIG. 11B. On the other hand, since both first opposite wall portions 10 of the external cylinder 9 have been pressed, the pressing pawls 14 abut against the front side wall of the annular convex portion 6 of the cylindrical member 4 of the male terminal portion 2 to push out the male terminal portion 2 in the direction
in which it slips out of the female terminal portion as shown in FIG. 10B. This allows the male terminal portion 2 to be easily disconnected from the female terminal portion 3.

As described above, with the one touch connector 1' for a tracheotomy tube according to the second embodiment, simply by rotating the regulating member 16 after the male terminal portion 2 and the female terminal portion 3 have been connected together so that the former is inserted into the latter, the regulating portions 17 prevent the fixture of the first opposite wall portions 10. This enables the maintenance of a connected state in which the male terminal portion 2 is surely prevented from slipping out of the female terminal portion 3. Furthermore, if the regulating member 16 is rotated to position each deregulating portion 18 inside the first opposite wall portion 10, then simply by pressing the first opposite wall portions 10 in the direction in which they come near each other, the locking pawls 15 are unlocked from the annular convex portion 6, while the pressing pawls 14 simultaneously push out the male terminal portion 2 via the annular convex portion 6. This loosens the tight engagement to allow the female terminal portion 3 to be smoothly disconnected from the male terminal portion 2. Also in this case, operations of rotating the regulating member 16 and pressing both first opposite side walls 10 can be performed with one hand. Therefore, it is possible to smoothly and promptly accomplish the connection of the tracheotomy tube A and the circuit tube B together and the removal of the circuit tube B from the tracheotomy tube A.

## Claims

1. A one touch connector assembly (1) for a tracheotomy tube which connector assembly comprises a male terminal portion (2) for being provided at a leading end of the tracheotomy tube and a female terminal portion (3) for being provided at a leading end of a respiration circuit terminal,
wherein said male terminal portion (2) comprises a tapered outer peripheral engagement surface (5) formed around an outer periphery of the male terminal portion (2) and an annular convex portion (6) projecting like a flange along a trailing edge of the outer peripheral engagement surface (5).
said female terminal portion (3) comprises a tapered inner peripheral engagement surface (7) formed around an inner periphery of the female terminal portion and engaged in pressure contact with said outer peripheral engagement surface (5), **characterized in that**
the female terminal portion (3) has an external cylinder (9) installed around an outer periphery of the female terminal portion (3) and formed of an elastic and flexible material,
the external cylinder (9) comprises a pair of first opposite wall portions (10) that lie opposite each other via the female terminal portion (3) and at a predetermined distance from said female terminal portion (3), and a pair of second opposite wall portions (11) connected to the respective first opposite wall portions (10) so as to be connected to the first opposite wall portions (10) and lying opposite each other via the female terminal portion (3) and close to said female terminal portion (3), the pair of second opposite wall portions (11) being snappily flexed and separated from the female terminal portion (3) when both first opposite wall portions (10) are pressed in a direction in which the first opposite wall portions (10) come near each other,
each of the second opposite wall portions (11) comprises a locking pawl (15) projected from an inner surface of the second opposite wall portion (11) and being locked on a rear side wall of the annular convex portion when said outer peripheral engagement surface (5) is engaged in pressure contact with said inner peripheral engagement surface (7), the locking pawl (15) being unlocked from the rear side wall of the annular convex portion when both first opposite wall portions (10) are pressed to snappily flex the second opposite wall portions (11), and
each of the first opposite wall portions (10) comprises a pressing pawl (14) that is projected from an inner surface of the first opposite wall portions (10) and abuts against a front side wall of said annular convex portion (6) when said pressing operation is performed, to press said male terminal portion (2) via the annular convex portion (6) in a direction in which said male terminal portion (2) slips out, thus disengaging said outer peripheral engagement surface (5) from said inner peripheral engagement surface (7).

2. The one touch connector for a tracheotomy tube according to claim 1, wherein said external cylinder comprises a fitting portion around an inner periphery of the external cylinder, the fitting portion being rotatably fitted into a groove portion formed all along the outer periphery of said female terminal portion.

3. The one touch connector for a tracheotomy tube according to claim 1 or 2, further comprising a regulating member which is removably provided between the outer periphery of said female terminal portion and each first opposite wall portion and which, when inserted between the outer periphery of the female terminal portion and each first opposite wall portion, regulates the flexure of both first opposite wall portions in which the first opposite wall portions come near each other, while maintaining the locking of said locking pawls on said annular convex portion.

4. The one touch connector for a tracheotomy tube according to claim 3, wherein said regulating member is formed like rings and rotatably installed on the outer periphery of said female terminal portion, and comprises: a pair of regulating portions being formed to be thick enough to regulate the flexure of both first opposite wall portions in the direction in which the first opposite wall portions come near each other, when the regulating member is rotated in one direction to insert each regulating portion between the outer periphery of the female terminal portion and the corresponding first opposite wall portion; and deregulating portions each connected to both regulating portions so as to be connected to the regulating portions and formed to be thinner than the regulating portions, the deregulating portions allowing both first opposite wall portions to freely flex in the direction in which the first opposite wall portions come near each other, when the regulating member is rotated in the other direction to place each deregulating portion between the outer periphery of the female terminal portion and the corresponding first opposite wall portion.

## Patentansprüche

1. Schnellverschluss-Verbindungsbaugruppe (1) für einen Luftröhrenkatheter, wobei die Verbindungsbaugruppe einen männlichen Endabschnitt (2) umfasst, der an einem Vorderende des Luftröhrenkatheters anzuordnen ist, und einen weiblichen Endabschnitt (3), der an einem Vorderende eines Beatmungsgerät-Anschlusses anzuordnen ist, wobei
der männliche Endabschnitt (2) eine konische äußere Randeingriffsfläche (5) umfasst, die um den Außenrand des männlichen Endabschnitts (2) herum ausgebildet ist, und einen ringförmigen konvexen Abschnitt (6), der wie ein Flansch entlang einer hinteren Kante der äußeren Randeingriffsfläche (5) herausragt,
der weibliche Endabschnitt (3) eine konische innere Randeingriffsfläche (7) umfasst, die um einen Innenrand des weiblichen Endabschnitts herum ausgebildet ist und mit Druck in die äußere Randeingriffsfläche (5) eingreift, **dadurch gekennzeichnet, dass**
der weibliche Endabschnitt (3) einen externen Zylinder (9) aufweist, der um den Außenrand des weiblichen Endabschnitts (3) herum eingebaut und aus einem elastischen und flexiblen Material ausgebildet ist,
der externe Zylinder (9) ein erstes Paar gegenüberliegende Wandabschnitte (10) umfasst, die einander bezogen auf den weiblichen Endabschnitt (3) gegenüberliegen und eine vorbestimmte Entfernung vom weiblichen Endabschnitt (3) haben, und ein zweites Paar gegenüberliegende Wandabschnitte (11), die so mit den jeweiligen ersten gegenüberliegenden Wandabschnitten (10) verbunden sind, dass sie mit den ersten gegenüberliegenden Wandabschnitten (10) verbunden sind und einander bezogen auf den weiblichen Endabschnitt (3) gegenüberliegen und sich nahe an dem Endabschnitt (3) befinden, wobei das Paar zweite gegenüberliegende Wandabschnitte (11) rasch gebogen und von dem weiblichen Endabschnitt (3) gelöst wird, wenn die beiden ersten gegenüberliegenden Wandabschnitte (10) in eine Richtung gedrückt werden, in der sich die ersten gegenüberliegenden Wandabschnitte (10) nahe kommen,
jeder der zweiten gegenüberliegenden Wandabschnitte (11) eine Sperrklinke (15) umfasst, die aus einer Innenseite des zweiten gegenüberliegenden Wandabschnitts (11) herausragt und in einer rückwärtigen Seitenwand des ringförmigen konvexen Abschnitts einrastet, wenn die äußere Randeingriffsfläche (5) gegen die innere Randeingriffsfläche (7) gedrückt wird, und sich die Sperrklinke (15) von der rückwärtigen Seitenwand des ringförmigen konvexen Abschnitts löst, wenn auf die beiden ersten gegenüberliegenden Wandabschnitte (10) gedrückt wird, damit sie die zweiten gegenüberliegenden Wandabschnitte (11) rasch biegen, und
jeder der ersten gegenüberliegenden Wandabschnitte (10) eine Druckklinke (14) aufweist, die aus einer Innenfläche der ersten gegenüberliegenden Wandabschnitte (10) vorsteht und gegen eine Vorderwand des ringförmigen konvexen Abschnitts (6) stößt, wenn Druck ausgeübt wird, damit der männliche Endabschnitt (2) über den ringförmigen konvexen Abschnitt (6) in eine Richtung gedrückt wird, in der der männliche Endabschnitt (2) herausrutscht und **dadurch** die äußere Randeingriffsfläche (5) von der inneren Randeingriffsfläche (7) löst.

2. Schnellverschluss-Verbindungsbaugruppe für einen Luftröhrenkatheter nach Anspruch 1, wobei der externe Zylinder einen Einsetzabschnitt umfasst, der einen Innenrand des externen Zylinders umgibt, und der Einsetzabschnitt drehend in einen Nutabschnitt eingesetzt wird, der entlang des gesamten Außenrands des weiblichen Endabschnitts ausgebildet ist.

3. Schnellverschluss-Verbindungsbaugruppe für einen Luftröhrenkatheter nach Anspruch 1 oder 2, zudem umfassend ein Regulierteil, das abnehmbar zwischen dem Außenrand des weiblichen Endabschnitts und jedem ersten gegenüberliegenden Wandabschnitt bereitgestellt ist, das beim Einsetzen zwischen den Außenrand des weiblichen Endabschnitts und jeden ersten gegenüberliegenden Wandabschnitt die Biegsamkeit der beiden ersten gegenüberliegenden Wandabschnitte reguliert, in denen sich die ersten gegenüberliegenden Wandabschnitte einander annähern, wobei die Sperrklinken in den ringförmigen konvexen Abschnitt eingerastet bleiben.

4. Schnellverschluss-Verbindungsbaugruppe für einen Luftröhrenkatheter nach Anspruch 3, wobei das Regulierteil ringartig geformt ist und drehbar auf dem Außenrand des weiblichen Endabschnitts angebracht ist, und umfasst: ein Paar Regulierabschnitte, die ausreichend dick ausgebildet sind, so dass sie die Biegsamkeit der beiden ersten gegenüberliegenden Wandabschnitte in der Richtung regulieren können, in der sich die ersten gegenüberliegenden Wandabschnitte einander annähern, wenn das Regulierteil in eine Richtung zum Einsetzen eines jeden Regulierteils zwischen den Außenrand des weiblichen Endabschnitts und den entsprechenden ersten gegenüberliegenden Wandabschnitt gedreht wird; und Deregulierteile, die jeweils mit beiden Regulierteilen verbunden sind, damit sie mit den Regulierteilen verbunden sind, und die dünner als die Regulierteile ausgebildet sind, wobei es die Deregulierteile den beiden ersten gegenüberliegenden Wandabschnitten ermöglichen, sich frei in der Richtung zu biegen, in der sich die ersten gegenüberliegenden Wandabschnitte einander annähern, wenn das Regulierteil in die andere Richtung gedreht wird, damit jedes Deregulierteil zwischen dem Außenrand des weiblichen Endabschnitts und dem entsprechenden ersten gegenüberliegenden Wandabschnitt angeordnet wird.

## Revendications

1. Ensemble de connecteur à une touche (1) pour tube de trachéotomie, lequel ensemble de connecteur comprend une partie terminale mâle (2) destinée à être placée à une extrémité avant du tube de trachéotomie et une partie terminale femelle (3) destinée à être placée à une extrémité avant d'un terminal de circuit de respiration,
dans lequel ladite partie terminale mâle (2) comprend une surface d'engagement périphérique extérieure rétrécie (5) formée autour d'une périphérie extérieure de la partie terminale mâle (2) et une partie convexe annulaire (6) faisant saillie comme une bride le long d'un bord de fuite de la surface d'engagement périphérique extérieure (5),
ladite partie terminale femelle (3) comprend une surface d'engagement périphérique intérieure rétrécie (7) formée autour d'une périphérie intérieure de la partie terminale femelle et engagée en contact sous pression avec ladite surface d'engagement périphérique extérieure (5),
**caractérisé en ce que**
la partie terminale femelle (3) comporte un cylindre extérieur (9) installé autour d'une périphérie extérieure de la partie terminale femelle (3) et fait d'un matériau élastique et souple,
le cylindre extérieur (9) comprend une paire de premières parties de paroi opposées (10) qui se trouvent l'une en face de l'autre via la partie terminale femelle (3) et à une distance prédéterminée de ladite partie terminale femelle (3), et une paire de deuxièmes parties de paroi opposées (11) connectées aux premières parties de paroi opposées respectives (10) de manière à être connectées aux premières parties de paroi opposées (10) et situées l'une en face de l'autre via la partie terminale femelle (3) et près de ladite partie terminale femelle (3), les deux deuxièmes parties de paroi opposées (11) étant fléchies et séparées par pression de la partie terminale femelle (3) lorsque les deux premières parties de paroi opposées (10) sont pressées dans une direction dans laquelle les premières parties de paroi opposées (10) se rapprochent l'une de l'autre,
chacune des deuxièmes parties de paroi opposées (11) comprend une griffe de verrouillage (15) qui fait saillie depuis une surface intérieure de la deuxième partie de paroi opposée (11) et qui est bloquée sur une paroi de côté arrière de la partie convexe annulaire lorsque ladite surface d'engagement périphérique extérieure (5) est engagée en contact sous pression avec ladite surface d'engagement périphérique intérieure (7), la griffe de verrouillage (15) étant dégagée de la paroi de côté arrière de la partie convexe annulaire lorsque les deux premières parties de paroi opposées (10) sont pressées pour fléchir par pression les deuxièmes parties de paroi opposées (11) ; et
chacune des premières parties de paroi opposées (10) comprend une griffe de pression (14) qui fait saillie depuis une surface intérieure des premières parties de paroi opposées (10) et qui bute contre une paroi de côté avant de ladite partie convexe annulaire (6) lorsque ladite opération de pressage est exécutée, pour presser ladite partie terminale mâle (2) via la partie convexe annulaire (6) dans une direction dans laquelle ladite partie terminale mâle (2) sort en glissant, en dégageant de ce fait ladite surface d'engagement périphérique extérieure (5) de ladite surface d'engagement périphérique intérieure (7).

2. Connecteur à une touche pour tube de trachéotomie selon la revendication 1, dans lequel ledit cylindre extérieur comprend une partie de raccord autour d'une périphérie intérieure du cylindre extérieur, la partie de raccord étant montée à rotation dans une partie rainure formée tout le long de la périphérie extérieure de ladite partie terminale femelle.

3. Connecteur à une touche pour tube de trachéotomie selon la revendication 1 ou 2, comprenant en outre un élément de régulation qui est placé de manière amovible entre la périphérie extérieure de ladite partie terminale femelle et chaque première partie de paroi opposée et qui, lorsqu'il est inséré entre la périphérie extérieure de la partie terminale femelle et chaque première partie de paroi opposée, régule la flexion des deux premières parties de paroi opposées dans laquelle les premières parties de paroi opposées se rapprochent l'une de l'autre, tout en maintenant le verrouillage desdites griffes de verrouillage sur ladite partie annulaire convexe.

4. Connecteur à une touche pour tube de trachéotomie selon la revendication 3, dans lequel ledit élément de régulation se présente sous la forme d'anneaux et est installé à rotation sur la périphérie extérieure de ladite partie terminale femelle, et comprend : une paire de parties régulatrices suffisamment épaisses pour réguler la flexion des deux premières parties de paroi opposées dans la direction dans laquelle les premières parties de paroi opposées se rapprochent l'une de l'autre, lorsque l'élément de régulation tourne dans une direction pour insérer chaque partie régulatrice entre la périphérie extérieure de la partie terminale femelle et la première partie de paroi opposée correspondante, et des parties dérégulatrices connectées chacune aux deux parties régulatrices afin d'être connectées aux parties régulatrices et plus minces que les parties régulatrices, les parties dérégulatrices permettant aux deux premières parties de paroi opposées de fléchir librement dans la direction dans laquelle les premières parties de paroi opposées se rapprochent l'une de l'autre, lorsque l'élément de régulation tourne dans l'autre direction pour placer chaque partie dérégulatrice entre la périphérie extérieure de la partie terminale femelle et la première partie de paroi opposée correspondante.
